# EUROPEAN PATENT APPLICATION

(11) **EP 4 282 454 A1**
(43) Date of publication of application: **29.11.2023**
(21) Application number: 22382508.4
(22) Date of filing: 26.05.2022
(51) Int. Cl.: A61M 16/06, A61M 16/08

(54) **PACIFIER FOR SUPPLYING A BREATHABLE GAS TO THE NOSTRILS OF AN INFANT**

(71) Applicant: Fundació Institut d'Investigació en Ciències de la Salut Germans Trias i Pujol, 08916 Badalona, Barcelona (ES)
(72) Inventor: DIAZ SOUTO, Rosana, 08029 BARCELONA (ES)
(74) Representative: ZBM Patents - Zea, Barlocci & Markvardsen

(57) **Abstract**

A pacifier (1) for supplying a breathable gas to the nostrils of an infant, comprising
- a mouth shield (2),
- a nipple (3) protruding from a front side of the mouth shield (2), and
- a support device (7) adapted for assembling a gas guide (4) with the mouth shield (2),
wherein the support device (7) comprises
- a plate (8) provided with a fastening surface (9) configured to be attached to a back side of the mouth shield (2); and
- an engaging portion (10) with a hook (11) adapted to retain by pressure the gas guide (4) in an operative position in use.

The support device (7) provides a simple and compact configuration, thus ensuring an easier assembly with the mouth shield (2), as well as a reliable retention of the gas guide (4) in the operative position. The pacifier (1) is comfortable to be used for infants.

## Description

The present disclosure relates to a pacifier for supplying a breathable gas to the nostrils of an infant.

The present disclosure also relates to a support device for assembling a gas guide to a pacifier.

### BACKGROUND

The use of a gas mask covering nose and mouth or the use of a nasal gas guide for assisting the breathing of an adult or infant patient is typically known, especially when the need exists of supplying a breathable gas for a long time, e.g. oxygen, inhaled medication, anesthesia or the like, according to a medical diagnostic. This occurs in many cases when the patient suffers from breathing diseases, nasal congestion, cold, after a medical operation, and the like.

Commonly, a nasal gas guide is provided with a pair of cannulas joined to each other by a bridge, wherein the pair of cannulas are provided with respective outlet conduits with an end configured to be inserted into the nostrils of a patient for delivering the breathable gas to be inhaled and provided with respective inlet conduits detachably coupled to a breathable gas source.

However, in practice these known breathing assistance devices may present some problems since they result in being uncomfortable to wear for infants, especially for neonates or premature babies. Moreover, the risk exists that the infant may remove the mask or the cannulas or even that the mask or the cannulas are not properly adjusted in place when the infant changes his or her position.

To address this problem, some pacifiers or soothers have been proposed that are provided with a pair of cannulas for delivering a breathable gas, e.g. oxygen or medication to be inhaled while the pacifier is inserted into the mouth for soothing the infant.

For example, US5375593 discloses a pacifier having a nipple, a mouthplate, a housing assembly and a pair of nasal cannulas coupled to one another and extending out of the housing so that whenever the infant has the nipple in his or her mouth the nasal cannulas are automatically directed to each nostril of the infant. A provision is made for connection of the nasal cannulas to an external oxygen source. In one embodiment a bladder is interposed between the nasal cannulas and the external oxygen source to ensure that even distribution of oxygen from the external source is provided to each nostril.

It is noted that such a pacifier requires a bulky configuration, wherein the housing assembly is joined to the mouth shield forming an integral unit.

In another example, WO2011137905 discloses a soother comprising a mouth shield, a nipple to be sucked on when placed in the mouth of the child, a fluid guide adapted for guiding a fluid, e.g. oxygen to be inhaled by a child, where the fluid guide comprises first and second fluid outlets for delivering the fluid to be inhaled and first and second fluids inlets connectable with a fluid delivering source, where the first outlet and the first inlet are formed as a first conduit, the second outlet and the second inlet are formed as a second conduit, and where the first and second conduits are connected by a connector integrally formed with the inlets and the outlets. The fluid guide is engageable with the back side of the mouth shield by means of a locking part configured to fix the connector of the fluid guide to the mouth shield.

In this case, the soother requires a complex structural configuration of the assembled parts in order to provide a reliable fixation of the fluid guide with the locking part onto the mouth shield.

Thus, there is a need to provide an improved pacifier for supplying a breathable gas, e.g oxygen or medication to the nostrils of an infant while the infant is sucking on the nipple of the pacifier, such that the gas guide is more easily assembled with the mouth shield of the pacifier in a suitable position, and having a simple structural configuration, thus reducing the number of pieces and the manufacturing costs of the pacifier.

It is also desirable to provide a support device for assembling a gas guide to a pacifier for assisting the breathing of an infant, with a simple and compact configuration, wherein the support device can be easily attached to the mouth shield of any conventional pacifier, and allows retaining a gas guide in the operative position when needed.

### SUMMARY

According to one aspect of the present disclosure, a pacifier for supplying a breathable gas to the nostrils of an infant is provided. The pacifier comprises a mouth shield, a nipple protruding from a front side of the mouth shield, and a support device adapted for assembling a gas guide with the mouth shield.

The support device comprises a plate provided with a fastening surface configured to be attached to a back side of the mouth shield; and an engaging portion with a hook adapted to retain by pressure the gas guide in an operative position in use.

Thanks to the simple configuration of the support device, an improved pacifier for assisting the breathing of an infant is obtained, in comparison with known pacifiers of the prior art. On the one hand, the engaging portion of the support device may be designed to provide a reliable retention of the gas guide in an optimal position for delivering the breathable gas to the nostrils to the infant. On the other hand, the fastening surface simplifies the fixation of the support device with the mouth shield of any conventional pacifier, thus providing more flexibility to choose a specific pacifier the user may prefer for the infant. In particular, the support device of the present disclosure is also suitable for neonates which usually use small size pacifiers with different shapes.

It is noted that the engaging portion may be configured so that the gas guide may be detachable coupled in a simple manner, thus enabling the same gas guide to be reused with another pacifier of the present disclosure. Likewise, the fastening surface may be configured to be detachable attached.

Therefore, the present pacifier is easier to be assembled by healthcare personnel thanks to the simple and compact configuration of the support device, enabling the gas guide to be reliably retained in the operative position meanwhile the infant is sucking the nipple of the pacifier, thus providing a comfortable use for the infants.

Further, since the present pacifier is configured with a fewer number of pieces to be coupled, the manufacturing costs thereof are also reduced.

In the present application, a "hook" is understood as a type of bracket to hold an object firmly in place by pressure, for example a C-shaped bracket, partly cut with an insertion opening and with enough flexibility to allow the insertion of the object to be hold.

According to one example of the present disclosure, the plate of the support device may be configured as an arc-shaped flat body with two legs and an upper edge, and the engaging portion may extend from the upper edge of the plate towards an opposite direction with respect to the fastening surface.

The fastening surface of the plate may comprise an adhesive layer covered by a peelable leaf.

In one example, the gas guide may comprise a pair of cannulas joined to each other by a bridge, the pair of cannulas being provided with two respective upper outlet conduits for delivering the gas to be inhaled and provided with two respective lower inlets conduits detachably coupled to a breathable gas source.

The hook of the engaging portion may be configured as a C-shaped bracket with an internal diameter which may be dimensioned to correspond with an external diameter of the bridge of a commercially available gas guide.

According to another aspect, the present disclosure also provides a support device for assembling a gas guide to a pacifier, as disclosed herein, for supplying a breathable gas to the nostrils of an infant. The support device comprises a plate provided with a fastening surface configured to be attached to a back side of the mouth shield, and an engaging portion configured with a hook adapted to retain by pressure the gas guide in an operative position in use.

In one preferred example, the support device is provided wherein:
- the plate is configured as an arc-shaped flat body with two legs and an upper edge, and wherein the engaging portion extends from the upper edge of the plate towards an opposite direction with respect to the fastening surface;
- the engaging portion comprises the hook adapted to retain by pressure the gas guide in the operative position; and
- the fastening surface of the plate comprises an adhered layer covered by a peelable leaf.

Thus, a support device for assembling a gas guide to a pacifier for assisting the breathing of an infant is obtained, with a simple and compact configuration, wherein the support device can be detachably engaged in an easy manner to the mouth shield of any conventional pacifier by means the adhesive layer, and wherein the hook facilitates a detachable retention of the gas guide in the operative position.

### BRIEF DESCRIPTION OF THE DRAWINGS

Non-limiting examples of the present disclosure will be described in the following, with reference to the appended drawings, in which:
Figure 1 is a perspective exploded view of a pacifier showing a gas guide and a support device according to one example of the present disclosure;
Figure 2 is a perspective view showing the gas guide and the support device assembled with the pacifier of figure 1, in the operative position in use;
Figure 3 is a perspective view of the support device of figures 1 and 2, showing the fastening surface without the peelable leaf;
Figure 4 is a perspective view of the support device of figure 3, showing the fastening surface with the peelable leaf;
Figure 5 is a frontal elevational view of the support device in figures 1 to 4;
Figure 6 is a lateral elevational view of the support device in figures 1 to 5;
Figure 7 is a top plan view of the support device in figures 1 to 6; and
Figure 8 is an elevational sectional view of the support device in figures 1 to 7 taken along line VIII-VIII in figure 7.

### DETAILED DESCRIPTION OF EXAMPLES

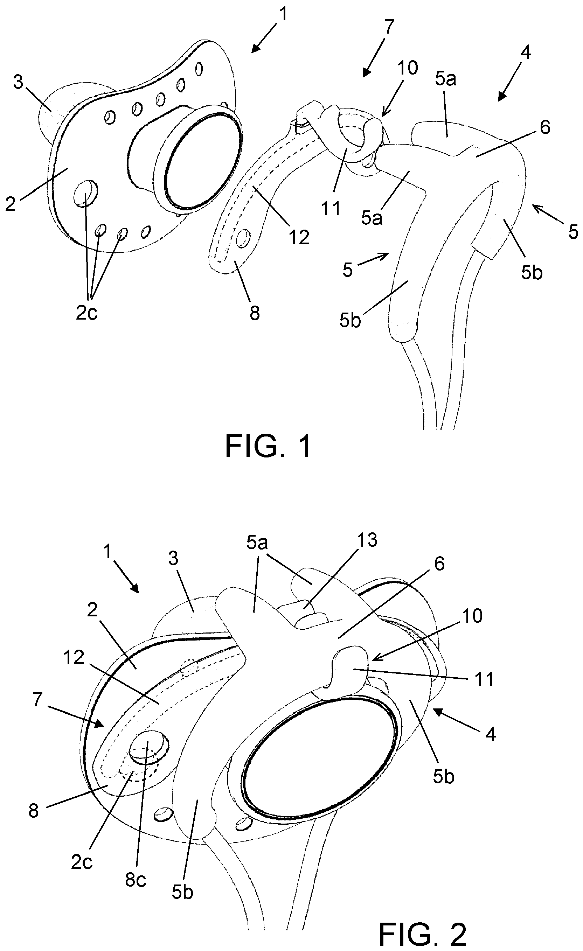

An example of the pacifier 1 of the present disclosure for supplying a breathable gas, e.g. oxygen, to the nostrils of an infant, can be show in figures 1 and 2. The present pacifier 1 comprises a mouth shield 2, a nipple 3 protruding from a front side of the mouth shield 2, and a support device 7 adapted for assembling a gas guide 4 with the mouth shield 2.

In the described example, the gas guide 4 comprises a pair of cannulas 5 joined to each other by a bridge 6. The pair of cannulas 5 are provided with respective two upper outlet conduits 5a for delivering the gas to be inhaled and provided with respective two lower inlets conduits 5b detachably coupled to a breathable gas source (not shown).

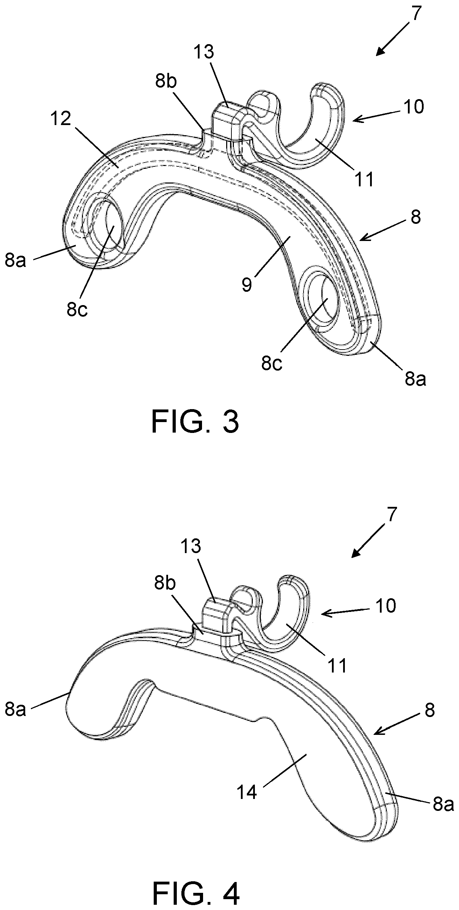

The support device 7 comprises a plate 8 provided with a fastening surface 9 (see figure 3) configured to be attached to a back side of the mouth shield 2; and an engaging portion 10 with a hook 11 adapted to retain by pressure the gas guide 4 in an operative position in use (see figure 2), as will be described in more detail below.

The support device 7 is shown in more detail in figures 3 to 8. In the present example, the plate 8 of the support device 7 is configured as an arc-shaped flat body with two legs 8a and an upper edge 8b, and the engaging portion 10 extends from the upper edge 8b of the plate 8 towards an opposite direction with respect to the fastening surface 9.

The described arc-shaped flat body is an optimal design to provide a compact and lightweight plate, so that the contour of the plate 8 does not extend from the contour of the mouth shield 2 of a conventional pacifier, thus preventing the plate 8 from touching the face of the infant.

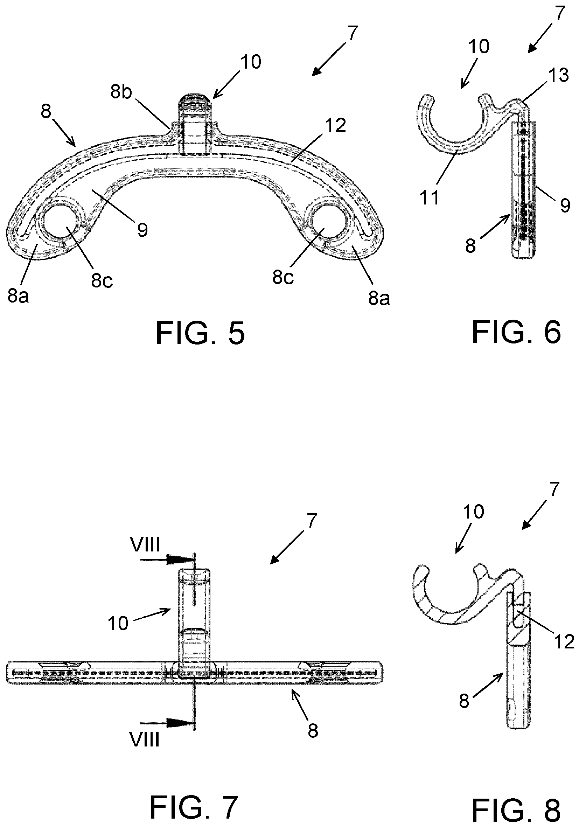

In the present example, the support device 7 is manufactured in two pieces (see for example figures 3 and 8), so that a first piece is made by an internal rib 12 (illustrated with dotted lines) and the engaging portion 10, wherein the internal rib 12 and the engaging portion 10 are integrally formed by a single piece; and so that a second piece is made by an over-molded plastic material enclosing the internal rib 12 embedded therein, thus conforming the plate 8.

The second piece of the support device 7 may be made of a hypoallergenic material, such as biomedical silicone. The first piece of the support device 7 may be made of a material that is more rigid than the second piece, such as ABS plastic (Acrylonitrile Butadiene Styrene), preferably ABS v0 since is an optimal material for the subsequent over-molding process.

It can be noted that the configuration of the support device 7 using two pieces as disclosed results in an optimal design which facilitates the manufacture by an over-molded process, and with the consequent reduction of the production costs.

In the described example, the hook 11 is configured as a C-shaped bracket (see for example figures 3, 6 and 8) with an internal diameter which may be dimensioned to correspond with an external diameter of the bridge 6 of the gas guide 4 as shown in figure 2, or even of another commercially available gas guide. However, other suitable shapes of the hook 11 can be considered to retain the gas guide 4 in a simple manner.

Further, the engaging portion 10 may be provided with a lip 13 contiguous to the hook 11 configured so that the lip 13 is fitted between both upper outlet conduits 5a of the pair of cannulas 5 once the gas guide 4 has been assembled in the operative position (see figure 2).

In the present example, the fastening surface 9 of the plate 8 comprises an adhesive layer covered by a peelable leaf 14, as can be seen in figure 4. This peelable leaf 14 is removable the first time before use, so that in the operative position the fastening surface 9 is adhered to the back side of the mouth shield 2. The adhered layer may be a double-sided adhesive tape, preferably made of rubber or polyethylene.

The disclosed configuration of the support device 7 with the C-shaped bracket 11 provides a simple and reliable fixation of the bridge 6 of the gas guide 4. Furthermore, the use of an adhesive layer simplifies the assembling of the support device 7 to the mouth shield 2 of the pacifier 1.

It is noted that for complying with safety regulations, the known pacifiers are required to have through holes 2c made in the mouth shield 2 (see figure 1) in order to form an airway, thus avoiding asphyxiation in case of any accidental swallowing of the pacifier 1. In this sense, the plate 8 of the support device 7 comprises at least a through hole 8c made in a position to at least partially overlap a respective through hole 2c of the mouth shield 2 (see figure 2). In the present example, the plate 8 includes two through holes 8c in the respective two legs 8a (see figures 3 and 5).

In the present example as shown in figures 1 and 2, the pair of cannulas 5 of the gas guide 4 may be configured so that the two lower inlet conduits 5b connected to the bridge 6 are arranged in a same plane substantially parallel to the mouth shield 2 in the operative position; and the two upper outlet conduits 5a connected to the bridge 6 are arranged in a same plane substantially perpendicular to the mouth shield 2 in the operative position, where the two upper outlet conduits 5a are oriented to the nostrils of the infant.

Further, the gas guide 4 may be made of a single tubular piece, with the pair of cannulas 5 and the bridge 6 in fluid communication (see also figures 1 and 2).

It is foreseen to provide different sizes for the support device 7 and the gas guide 4 depending on the age of the infant, particularly for neonates or premature babies, in order to obtain a reliable fixation of the bridge 6 of gas guide 4 with the hook 11 of the support device 7, preventing the gas flow from being throttled.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow. If reference signs related to drawings are placed in parentheses in a claim, they are solely for attempting to increase the intelligibility of the claim, and shall not be construed as limiting the scope of the claim.

## Claims

1. A pacifier (1) for supplying a breathable gas to the nostrils of an infant, comprising
- a mouth shield (2),
- a nipple (3) protruding from a front side of the mouth shield (2), and
- a support device (7) adapted for assembling a gas guide (4) with the mouth shield (2),
wherein the support device (7) comprises
- a plate (8) provided with a fastening surface (9) configured to be attached to a back side of the mouth shield (2); and
- an engaging portion (10) with a hook (11) adapted to retain by pressure the gas guide (4) in an operative position in use.

2. The pacifier (1) according to claim 1, wherein the plate (8) of the support device (7) is configured as an arc-shaped flat body with two legs (8a) and an upper edge (8b), and wherein the engaging portion (10) extends from the upper edge (8b) of the plate (8) towards an opposite direction with respect to the fastening surface (9).

3. The pacifier (1) according to claim 2, wherein the support device (7) is manufactured in two pieces, so that
- a first piece is made by an internal rib (12) and the engaging portion (10), wherein the internal rib (12) and the engaging portion (10) are integrally formed by a single piece, and
- a second piece is made by an over-molded plastic material enclosing the internal rib (12) embedded therein, thus conforming the plate (8).

4. The pacifier (1) according to claim 3, wherein the second piece of the support device (7) is made of a hypoallergenic material, such as biomedical silicone.

5. The pacifier (1) according to claim 3 or 4, wherein the first piece of the support device (7) is made of a material that is more rigid than the second piece, such as ABS plastic (Acrylonitrile Butadiene Styrene), preferably ABS v0.

6. The pacifier (1) according to any of the preceding claims, wherein the fastening surface (9) of the plate (8) comprises an adhesive layer covered by a peelable leaf (14).

7. The pacifier (1) according to claim 6, wherein the adhesive layer is a double-sided adhesive tape, preferably made of rubber or polyethylene.

8. The pacifier (1) according to any of the preceding claims, wherein the plate (8) comprises at least a through hole (8c) made in a position to at least partially overlap a respective through hole (2c) of the mouth shield (2).

9. The pacifier (1) according to any of the preceding claims, wherein the gas guide (4) comprises a pair of cannulas (5) joined to each other by a bridge (6), the pair of cannulas (5) being provided with two respective upper outlet conduits (5a) for delivering the gas to be inhaled and provided with two respective lower inlets conduits (5b) detachably coupled to a breathable gas source.

10. The pacifier (1) according to any preceding claims, wherein the hook (11) of the engaging portion (10) is configured as a C-shaped bracket.

11. The pacifier (1) according to claim 9 or 10, wherein the engaging portion (10) is provided with a lip (13) contiguous to the hook (11) configured so that the lip (13) is fitted between both upper outlet conduits (5a) of the pair of cannulas (5) once the gas guide (4) has been assembled in the operative position.

12. The pacifier (1) according to any of claims 9 to 11, wherein the gas guide (4) is made of a single tubular piece with the pair of cannulas (5) and the bridge (6) in fluid communication.

13. The pacifier (1) according to any of claims 10 to 12, wherein the pair of cannulas (5) of the gas guide (4) are configured so that
- the two lower inlet conduits (5b) connected to the bridge (6) are arranged in a same plane substantially parallel to the mouth shield (2) in the operative position, and
- the two upper outlet conduits (5a) connected to the bridge (6) are arranged in a same plane substantially perpendicular to the mouth shield (2) in the operative position, where the two upper outlet conduits (5a) are oriented to the nostrils of the infant.

14. A support device (7) for assembling a gas guide (4) to a pacifier (1) for supplying a breathable gas to the nostrils of an infant, wherein the support device (7) comprises
- a plate (8) provided with a fastening surface (9) configured to be attached to a back side of the mouth shield (2), and
- an engaging portion (10) with a hook (11) adapted to retain by pressure the gas guide (4) in an operative position in use.

15. The support device (7) according to claim 14, wherein
- the plate (8) is configured as an arc-shaped flat body with two legs (8a) and an upper edge (8b), and wherein the engaging portion (10) extends from the upper edge (8b) of the plate (8) towards an opposite direction with respect to the fastening surface (9);
- the engaging portion (10) comprises the hook (11) adapted to retain by pressure the gas guide (4) in the operative position; and
- the fastening surface (9) of the plate (8) comprises an adhesive layer covered by a peelable leaf (14).
